# EUROPEAN PATENT SPECIFICATION

(11) **EP 1 496 095 B1**
(45) Date of publication and mention of the grant of the patent: **20.12.2006**
(21) Application number: 03743532.8
(22) Date of filing: 28.02.2003
(51) Int. Cl.: C09K 3/00, C11B 9/00, A61K 9/06, A23L 1/226, A61Q 99/00, A61Q 5/00, A61Q 5/02, A61Q 11/00, A61Q 15/00, A61Q 19/00, A61Q 19/10

(54) **REFRIGERANT COMPOSITIONS AND USES THEREOF**
KÜHLENDE ZUSAMMENSETZUNGEN UND DEREN VERWENDUNGEN
COMPOSITIONS REFRIGERANTES ET LEURS UTILISATIONS

(30) Priority: 01.03.2002 JP 2002056407
(43) Date of publication of application: 12.01.2005
(73) Proprietor: Takasago International Corporation, Tokyo-to 144-8721 (JP)
(72) Inventor: ISHIDA, Kenya, Takasago International Corp., Hiratsuka-shi, Kanagawa 254-0073 (JP); SAKURAI, Kazutoshi, Takasago International Corp., Ohta-ku, Tokyo 144-8721 (JP)
(74) Representative: Reinhard - Skuhra - Weise & Partner
(86) International application number: PCT/JP2003/002368
(87) International publication number: WO 2003/074622

(56) References cited:
- EP-A- 1 149 820
- EP-A2- 0 988 852
- WO-A-02/13776
- WO-A-02/15692
- JP-A- 2 255 890
- JP-A- 7 215 848
- JP-A- 56 100 715
- JP-A- 2000 247 859
- JP-A- 2000 351 725
- PINO, J A, MARBOT R, FUENTES, V: "Characterisation of Volatiles in Bullock's Heart (Annona reticulata L.) Fruit Cultivars from Cuba" JOURNAL OF AGRICULTURAL AND FOOD CHEMISTRY, vol. 51, 20 May 2003 (2003-05-20), pages 3836-3839, XP002322766
- PATENT ABSTRACTS OF JAPAN vol. 2000, no. 04, 31 August 2000 (2000-08-31) & JP 2000 026257 A (KAO CORP), 25 January 2000 (2000-01-25)

## Description

### Technical Field

The present invention relates to a cool feeling composition capable of obtaining a long-lasting cool feeling effect when mixed with foods and drinks, cosmetics such as hair-care or body-care cosmetics, toiletry products, bath agents, pharmaceuticals, and the like. Particularly, it relates to a cool feeling composition capable of maintaining a cool feeling effect without impairing cool feeling resulting from 1-menthol and also capable of obtaining an effect of retaining more preferred smell through alleviation of a irritant mint odor into a mild odor. The invention further relates to the use of a composition for the preparation of a product for producing a cool feeling effect and capable of enhancing cool feeling strength of 1-menthol or the like and imparting a persistency. The invention still further relates to a fragrance composition and various products containing the same.

### Background Art

With the recent drastic changes in consumers' life style and needs, there arises a trend toward commonly used various products such as cosmetics, toiletry products, bath agents, and pharmaceuticals having not only the inherent functions requisite for the products but also an additional function of imparting a refresh feeling during or after use, and they have been commercially available in various commodity forms. Moreover, also in food and drink, some of them are desired to afford a refreshing feeling at eating and drinking or to retain a refreshing feeling even after eating and drinking.

Such products with a preference for imparting a refreshing feeling and a cool feeling effect are typified by cosmetics to be used in summer, toiletry goods such as shampoos, rinses, and conditioners, body-care goods such as body shampoos, cataplasms, bath agents, insect-repellent sprays, and the like.

To satisfy such requirement, various cool feeling agents such as 1-menthol, camphor, menthyl malonate, borneol, cineol, menthone, spearmint, peppermint, isopulegol, 3-(1-mentoxy)propane-1,2-diol, p-menthane-3,8-diol, and glycosyl monomenthyl-orthoacetate have hitherto been mixed with the products.

In order to correspond to the increasing demand on the market for these cool feeling agents, there have been intensively performed researches and developments to provide novel cool feeling agents hitherto unknown. Also, with regard to the cool feeling agents hitherto known, combination of two or more cool feeing substances have been made for the purpose of enhancing a cool feeling effect or improving a lasting effect of the cool feeling effect, and researches have been actively made to improve a cool feeling effect by combining cool feeling agents with other substances, to investigate compatibility of cool feeling agents with various ingredients in the products, and to enhance the cool feeling effect or improve a feeling in use when the cool feeling agents are mixed with the various products. In addition, alleviation of irritant odor resulting from menthol has been also investigated particularly in the case of using menthol.

Some examples of novel cool feeling agents or compositions exhibiting an improved cool feeling effect hitherto proposed include use of isopulegol as a cool feeling agent and use of the cool feeling agent in cosmetics, pharmaceuticals, and the like (JP-A-6-65023), use of 3-(1-menthoxy)propane-1,2-diol known as a cool feeling agent (JP-B-61-48813) together with a specific glyceryl ether as a cosmetic for summer (JP-A-63-208505), use of 3-(1-menthoxy)propane-1,2-diol together with a hydrophilic polyether-modified silicone as an aerosol for anti-inflammatory agents (JP-A-63-264522), and the like. JP-A-6-107527 proposes a hair wash composition wherein a known cool feeling agent such as menthol or camphor is combined with a specific warm feeling agent, which exhibits both of an excellent conditioning property and an excellent refreshing feeling. Although these compositions show a somewhat increased cool feeling effect, persistency of the cool feeling effect has not been investigated.

On the other hand, JP-A-2000-44924 proposes a cool feeling agent composition wherein a specific cool feeling agent including 1-menthol is combined with a specific vanillyl ether, but no investigation to alleviate irritation resulting from menthol has been made.

As mentioned above, although various investigations, e.g., the combinations of cool feeling agents themselves or cool feeling agents and other substances, various products using the same, and the like have been made, consumers have desired to develop those more excellent in persistency of a refreshing feeling in various products and also, particularly in the case of using menthol as a cool feeling agent, to provide products having a mild smell in which the irritant menthol odor is reduced.

### Disclosure of the Invention

An object of the invention is to provide a cool feeling composition, a use of a composition for the preparation of a product for producing a cool feeling effect, and a fragrance composition and various products containing the same, which satisfy the aforementioned properties hitherto required.

That is, an object of the invention is to provide a cool feeling composition capable of providing products exhibiting a high cool feeling effect and having an excellent persistency when mixed with various products and a blended fragrant material containing the cool feeling composition, and a food and drink, cosmetic, toiletry product, bath agent, pharmaceutical, and the like containing the above cool feeling composition or the blended fragrant material containing the cool feeling composition.

Moreover, an object of the invention is to provide a cool feeling composition capable of alleviating an irritant fragrant note of 1-menthol which has a cool feeling effect but a strong mint odor or which lacks persistency of the cool feeling effect owing to its subliming property, also capable of improving the strength and persistency of the cool feeling effect of the cool feeling agent, and having a more preferable refreshing feeling, a blended fragrant material containing the cool feeling composition, and a food and drink, cosmetic, toiletry product, bath agent, pharmaceutical, and the like containing the above cool feeling composition or the blended fragrant material containing the cool feeling composition.

Furthermore, an object of the invention is to provide use of a composition as specified thereafter for the preparation of product for producing a cool feeling effect in order to be capable of improving cool feeling strength and persistency of the cool feeling effect when combined with a cool feeling substance, particularly in the case of using menthol as a cool feeling agent, capable of alleviating an irritant fragrant note of 1-menthol, a blended fragrant material containing the cool feeling auxiliary composition together with 1-menthol, and a food and drink, cosmetic, toiletry product, bath agent, pharmaceutical, and the like containing 1-menthol and the cool feeling auxiliary composition or the blended fragrant material containing 1-menthol and the cool feeling auxiliary composition.

As a result of the extensive studies for achieving the above objects, the present inventors have found that use of a specific salicylic acid ester in combination with a specific cool feeling agent improves cool feeling strength, persistency and the like of a cool feeling composition and enables impartment of more preferred refreshing effect and also alleviation of an irritant odor resulting from 1-menthol in the case of using 1-menthol as a cool feeling agent, and further, in a fragrance composition containing the cool feeling composition, fragrance diffusivity and fragrance-remaining property of the fragrance composition are enhanced and a high fragrance-quality improving effect is imparted to products containing the fragrance composition. Based on these findings, they have accomplished the invention.

The invention relates to a cool feeling composition, which comprises a cool feeling substance comprising (A) 1-menthol and (B) at least one compound selected from isopulegol, 3-(1-menthoxy)propane-1,2-diol, 2-(1-menthoxy)ethane-1-ol, 3-(1-menthoxy)propane-1-ol, 2-methyl-3-(1-menthoxy)propane-1,2-diol, N-ethyl-1-menthylcarboxamide, p-menthane-3,8-diol, 1-menthyl lactate, and 2-isopropyl-N,2,3-trimethylbutanamide, and (C) a salicylic acid ester represented by the following general formula (I), composition ratios of the ingredient (A) to the ingredient (B), the ingredient (B) to the ingredient (C), and the ingredient (A) to the ingredient (C) by weight being 95:5 to 30:70, 90:10 to 30:70, and 99.5:0.5 to 30:70, respectively: 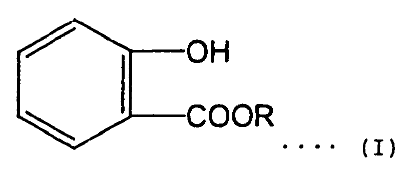 wherein R represents a 2-hydroxyethyl group, a 2,3-dihydroxypropyl group, a benzyl group, a phenethyl group or a menthyl group.

Additionally, the invention relates to the above cool feeling composition, wherein the cool feeling substances of the ingredient (B) are isopulegol and 3-(1-menthoxy)propane-1,2-diol, and the salicylic acid ester of the ingredient (C) is 2-hydroxyethyl salicylate.

In addition, the invention relates to the use of a composition, which comprises (B) at least one compound selected from isopulegol, 3-(1-menthoxy)propane-1,2-diol, 2-(1-menthoxy)ethane-1-ol, 3-(1-menthoxy)propane-1-ol, 2-methyl-3-(1-menthoxy)propane-1,2-diol, N-ethyl-1-menthylcarboxamide, p-menthane-3,8-diol, 1-menthyl lactate, 2-isopropyl-N,2,3-trimethylbutanamide, (C) at least one salicylic acid ester represented by the above general formula (I), and, according to necessity, (A) 1-menthol,
composition ratios of the ingredient (A) to the ingredient (B), the ingredient (B) to the ingredient (C), and the ingredient (A) to the ingredient (C) by weight being 0:100 to 20:80, 90:10 to 30:70, and 0:100 to 20:80, respectively, for the preparation of a product for producing a cool feeling effect.

This composition is refered to herein as "cool feeling auxiliary composition."

Moreover, the invention relates to a fragrance composition, wherein any one of the above cool feeling compositions is mixed in an amount of 0.01 to 90% by weight.

Furthermore, the invention relates to a food and drink, cosmetic, toiletry, bath agent, or pharmaceutical, wherein any one of the above cool feeling compositions is mixed in an amount of 0.001 to 20% by weight.

Also, the invention relates to a food and drink, cosmetic, toiletry, bath agent, or pharmaceutical, wherein 1-menthol and the above cool feeling composition are mixed.

Moreover, the invention relates to a food and drink, cosmetic, toiletry, bath agent, or pharmaceutical, which contains the above fragrance composition in an amount of 0.01 to 20% by weight.

### Best Mode for Carrying Out the Invention

In the invention, as the cool feeling substances, (A) 1-menthol, and (B) at least one compound selected from isopulegol, 3-(1-menthoxy)propane-1,2-diol, 2-(1-menthoxy)ethane-1-ol, 3-(1-menthoxy)propane-1-ol, 2-methyl-3-(1-menthoxy)propane-1,2-diol, N-ethyl-1-menthylcarboxamide, p-menthane-3,8-diol, 1-menthyl lactate, 2-isopropyl-N,2,3-trimethylbutanamide are used.

Moreover, in the salicylic acid ester (C) represented by the above general formula (I) to be incorporated into cool feeling compositions containing these cool feeling agents, R represents a 2-hydroxyethyl group, a 2,3-dihydroxypropyl group, a benzyl group, a phenethyl group, or a menthyl group. Particularly preferred is a 2-hydroxyethyl group. These salicylic acid esters represented by the general formula (I) can be produced by known methods. Moreover, 2-hydroxyethyl salicylate, 2,3-dihydroxypropyl salicylate, benzyl salicylate, phenethyl salicylate, and menthyl salicylate, which are compounds of the groups mentioned in claim 1 for R's, are all commercially easily available.

The salicylic acid esters are known as anti-inflammatory agents which are easily absorbed through skin, improve blood flow at an affected area, increase metabolism, and suppress inflammation and pain caused by muscular fatigue, bruise, sprain, and the like. Moreover, menthyl salicylate and 2-hydroxyethyl salicylate are known as UV absorbers. Furthermore, as compared with menthol, it is reported that methyl salicylate has a very weak stimulation of cool feeling and rather a strong stimulation of hot feeling (Journal of The Society of Cosmetic Chemist, 43, pp 131-147). Namely, with regard to the salicylic acid esters, not only cool feeling effect but also an effect of the combined use with the other cool feeling substances have been not at all known.

On the other hand, with regard to the cool feeling substance, heretofore, tonic shampoos using 1-menthol having a subliming characteristic have been widely known and the mixing amount of 1-menthol to be used therein is an amount of at least 0.5%. However, even when such amount is mixed, 1-menthol rapidly sublimed after use and hence the cool feeling lasts not so long. Also, in the case of using 1-menthol as a cool feeling substance, 1-menthol strongly imparts a virile image because irritation with a strong mint odor intensively affects eyes and skin.

Moreover, with regard to 1-menthol, cool feeling compositions together with isopulegol or 3-(1-menthoxy)propane-1,3-diol have been known but there remains a problem that, even when the compositions are employed in body-care products such as shampoo as they are, balance of smell is still poor and a mint odor characteristic to 1-menthol remains, whereby the smell is rather still limited to virile one.

The salicylic acid ester (C) for use in the invention not only can improve cool feeling strength of the cool feeling substance and persistency of the cool feeling effect thereof but also has an effect that the irritant odor of 1-menthol is alleviated. Therefore, the salicylic acid ester for use in the invention can exhibit a particularly excellent effect in the case of combination with 1-menthol among combinations with cool feeling substances. In addition, at that time, in view of improving the cool feeling strength of 1-menthol and persistency of the cool feeling effect thereof, 1-menthol is used in combination with the other cool feeling substance. Such other cool feeling substance includes cool feeling substances exemplified in the above excluding 1-menthol, i.e., isopulegol, 3-(1-menthoxy)propane-1,2-diol, 2-(1-menthoxy)ethane-1-ol, 3-(1-menthoxy)propane-1-ol, 2-methyl-3-(1-menthoxy)propane-1,2-diol, N-ethyl-1-menthylcarboxamide, p-menthane-3,8-diol, 1-menthyl lactate, and 2-isopropyl-N,2,3-trimethylbutanamide. These other cool feeling substances may be used, in combination with 1-menthol, solely or as a mixture of two or more of them.

As above, when 1-menthol is used as a cool feeling substance, by using it together with a salicylic acid ester represented by the general formula (I), a mild and balanced fragrance also preferred by women can be achieved and also the irritant odor of 1-menthol can be alleviated, as well as diffusivity of a refreshing smell, cool feeling strength, and persistency thereof can be further improved.

Moreover, when these cool feeling compositions are used together with fragrance compositions, fragrance diffusivity and fragrance-remaining property of the fragrances are enhanced and a similar high effect is imparted to the products containing the fragrance compositions.

Therefore, as the cool feeling composition of the invention, the compositions include a cool feeling composition which comprises (A) 1-menthol, (B) at least one compound selected from isopulegol, 3-(1-menthoxy)propane-1,2-diol, 2-(1-menthoxy)ethane-1-ol, 3-(1-menthoxy)propane-1-ol, 2-methyl-3-(1-menthoxy)propane-1,2-diol, N-ethyl-1-menthylcarboxamide, p-menthane-3,8-diol, 1-menthyl lactate, and 2-isopropyl-N,2,3-trimethylbutanamide, and (C) a salicylic acid ester represented by the above general formula (I). The composition ratio (weight ratio) of the ingredient (A) and the ingredient (B) and the ingredient (C) in the cool feeling composition is expressed by the composition ratios of the ingredient (A) to the ingredient (B), the ingredient (B) to the ingredient (C), and the ingredient (A) to the ingredient (C), which are in the range of 95:5 to 30:70, 90:10 to 30:70, and 99.5:0.5 to 30:70, respectively, preferably in the range of 90:10 to 50:50, 90:10 to 40:60, and 99:1 to 50:50, respectively.

In the ratio of the ingredient (A) to the ingredient (B), the effect of alleviating the irritant odor of the ingredient (A) is not so clear when the ingredient (A) exceeds 95%, and the cool feeling effect becomes weak when the ingredient (B) exceeds 70%. Moreover, in the ratio of the ingredient (B) to the ingredient (C), the cool feeling strength and the irritant odor-alleviating effect, particularly, the irritant odor-alleviating effect is not observed when the ingredient (B) exceeds 90%, and the cool feeling effect becomes weak when the ingredient (C) exceeds 70%. Furthermore, in the ratio of the ingredient (A) to the ingredient (C), the effect of alleviating the irritant odor of the ingredient (A) is hardly observed when the ingredient (A) exceeds 99.5%, and natural feeling and minty feeling on the fragrance of the cool feeling composition are remarkably decreased and the cool feeling effect becomes weak when the ingredient (C) exceeds 70%. In this connection, use of the ingredient (A) to the ingredient (B), the ingredient (B) to the salicylic acid ester (C), and the ingredient (A) to the ingredient (C) in the rage of 90:10 to 70:30, 90:10 to 40:60, and 99:1 to 80:20, respectively is extremely preferred in view of costs, the irritant odor-alleviating effect, the cool feeling strength, persistency, and the fragrance quality-improving effect of the fragrance composition.

Moreover, also in the combination of the above (A), (B), and (C), as the salicylic acid ester of the ingredient (C), preferred are 2-hydroxyethyl salicylate, 2,3-dihydroxypropyl salicylate, benzyl salicylate, phenethyl salicylate, and menthyl salicylate. Of these, 2-hydroxyethyl salicylate is more preferred in view of both of the cool feeling-enhancing and cool feeling-persisting effects and the fragrance diffusive and persisting effects of the fragrance composition. As the ingredient (B), isopulegol and 3-(1-menthoxy)propane-1,2-diol are suitably used in view of the above effects and particularly when the both compounds are used together, excellent effects are obtained in view of the cool feeling strength and the cool feeling persistency.

The adding amount of the cool feeling composition of the invention to a product varies depending on a usual amount per once at use of the product to be added, a use form of the product, i.e., whether the product is used as it is or is sprayed as an aerosol, a form of the product, i.e., whether it is liquid or solid, and the like. However, in general, the amount is preferably about 0.01 to 20% by weight, more preferably about 0.01 to 10% by weight based on the total amount of the product composition. The adding amount of the cool feeling composition to the product may be in an amount range where the effect(s) of the invention can be obtained, and there is no upper or lower limitation on the adding amount.

Moreover, the cool feeling composition of the invention may be mixed in a form of a composition at the time when it is mixed with a product or may be used as an embodiment wherein a main cool feeling substance and other substances in the cool feeling composition are separately added and mixed at production of the product so as to achieve the composition ratio of the cool feeling composition of the invention in the product. Also, the composition may be used as an embodiment wherein ingredients other than the main cool feeling substance or part of the main cool feeling substance and ingredients other than the main cool feeling substance are separately produced as a cool feeling auxiliary composition and this composition is mixed with the main cool feeling substance before use to produce the cool feeling composition of the invention or as an embodiment wherein the main cool feeling substance and the cool feeling auxiliary composition are separately added to the product so as to achieve the composition ratio of the cool feeling composition of the invention in the product. At this time, the cool feeling auxiliary composition functions as a composition to improve the cool feeling strength and persistency of the cool feeling effect of the main cool feeling substance and further as a composition to alleviate the irritant odor of 1-menthol. In the case that the main cool feeling substance is 1-menthol as an example, a preferred composition of the cool feeling auxiliary composition is a composition which comprises (A) 1-menthol, (B) at least one compound selected from isopulegol, 3-(1-menthoxy)propane-1,2-diol, 2-(1-menthoxy)ethane-1-ol, 3-(1-menthoxy)propane-1-ol, 2-methyl-3-(1-menthoxy)propane-1,2-diol, N-ethyl-1-menthylcarboxamide, p-menthane-3,8-diol, 1-menthyl lactate, and 2-isopropyl-N,2,3-trimethylbutanamide, and (C) a salicylic acid ester represented by the above general formula (I), composition ratios of the ingredient (A) to the ingredient (B), the ingredient (B) to the ingredient (C), and the ingredient (A) to the ingredient (C) by weight being 0:100 to 20:80, 90:10 to 30:70, and 0:100 to 20:80, respectively. In the case of the cool feeling auxiliary composition, the ingredient (A), i.e., 1-menthol is an optional ingredient.

Furthermore, in the invention, the above cool feeling composition is first mixed with a fragrance composition to form a cool feeling composition-containing fragrance composition (fragrance composition of the invention), and then the cool feeling composition-containing fragrance composition can be mixed with a product. At this time, the main cool feeling substance and the cool feeling auxiliary composition may be separately mixed with a fragrance composition. The fragrance note of the fragrance composition to be mixed with the cool feeling composition of the invention is preferably constituted by at least one or more selected from minty, citrus, herbal, green, floral, and marine notes, and the mixing amount of the cool feeling composition in the fragrance composition is generally 0.01 to 90% by weight, preferably 0.1 to 50% by weight.

As mentioned above, the fragrance composition of the invention contains a main cool feeling substance and a cool feeling auxiliary composition, i.e., a cool feeling composition. In the fragrance composition of the invention, as fragrance substances, ketones, aldehydes, esters, alcohols, ethers, terpenes, natural essential oils, synthetic musk, and other fragrant materials usually employed can be mixed in an appropriate combination.

In other words, the fragrant materials for use in the invention are not limited in kind and include those usually employed in various products, such as preparations for external application, e.g., cosmetics and pharmaceuticals, cleansers for human body, clothes, and hard surfaces, bleaches, and softeners. Specific examples thereof include synthetic fragrant materials, e.g., hydrocarbons such as aliphatic hydrocarbons, terpene hydrocarbons, and aromatic hydrocarbons; alcohols such as aliphatic alcohols, terpene alcohols, and aromatic alcohols; ethers such as aliphatic ethers and aromatic ethers; oxides such as aliphatic oxides and oxides of terpenes; aldehydes such as aliphatic aldehydes, terpene aldehydes, hydrogenated aromatic aldehydes, thioaldehydes, and aromatic aldehydes; ketones such as aliphatic ketones, terpene ketones, hydrogenated aromatic ketones, aliphatic cyclic ketones, non-benzenoid aromatic ketones, and aromatic ketones; acetals, ketals, phenols, phenol ethers; acids such as fatty acids, terpene acids, hydrogenated aromatic carboxylic acids, and aromatic carboxylic acids; acid amides; lactones such as aliphatic lactones, macrocyclic lactones, terpene lactones, hydrogenated aromatic lactones, and aromatic lactones; esters such as aliphatic esters, furan carboxylic esters, alicyclic carboxylic esters, cyclohexylcarboxylic esters, terpene carboxylic esters, and aromatic carboxylic esters; and nitrogen-containing compounds, such as nitromusks, nitriles, amines, pyridines, quinolines, pyrrole, and indole, and natural fragrant materials derived from animals or plants. Typical fragrant materials usable in the invention are listed below.

C6-C12 Aldehydes, anisaldehyde, acetal R, acetophenone, acetylcedrene, adoxal, allylamyl glycolate, allyl cyclohexanepropionate, alpha-damascone, beta-damascone, delta-damascone, ambrettolide, ambroxan, amylcinnamic aldehyde, amylcinnamic aldehyde dimethylacetal, amyl valerianate, amyl salicylate, isoamyl acetate, isoamyl salicylate, aurantiol, acetyl eugenol, bacdanol, benzyl acetate, benzyl alcohol, benzyl salicylate, bergamyl acetate, bornyl acetate, butyl butyrate, p-tert-butylcyclohexanol, p-tert-butylcyclohexyl acetate, o-tert-butylcyclohexanol, benzaldehyde, benzyl formate, caryophyllene, cashmerane, carvone, cedramber, cedryl acetate, cedrol, celestolide, cinnamic alcohol, cinnamic aldehyde, cis-jasmone, citral, citral dimethyl acetal, citrasal, citronellal, citronellol, citronellyl acetate, citronellyl formate, citronellyl nitrile, cyclaset, cyclamen aldehyde, cyclaprop, caron, coumarin, cinnamyl acetate, delta-C6-C13 lactones, dimethylbenzylcarbinol, dihydrojasmon, dihydrolinalool, dihydromyrcenol, dimetol, dimyrcetol, diphenyl oxide, ethyl vanillin, eugenol, fruitate, fenchyl alcohol, phenylethyl phenylacetate, galaxolide, gamma-C6-13 lactones, geraniol, geranyl acetate, geranyl formate, geranyl nitrile, hedion, helional, heliotropin, cis-3-hexenol, cis-3-hexenyl acetate, cis-3-hexenyl salicylate, hexylcinnamic aldehyde, hexyl salicylate, hyacinth dimethyl acetal, hydrotropic alcohol, hydroxycitronellal, indole, ionone, isobornyl acetate, isocyclocitral, Iso E Super, isoeugenol, isononyl acetate, isobutylquinoline, jasmal, jasmolactone, jasmophirane, corvone, ligustoral, lilial, lime oxide, limonene, linalool, linalool oxide, linalyl acetate, lyral, manzanate, myol, menthanyl acetate, menthonate, methyl anthranilate, methyl eugenol, menthol, alpha-methylionone, beta-methylionone, gamma-methylionone, methyl isoeugenol, methyl lavender ketone, methyl salicylate, muguet aldehyde, mugol, musk TM-II, musk 781, musk C14, musk T, musk ketone, musk tibetene, musk moskene, myrac aldehyde, methylphenyl acetate, nerol, neryl acetate, nopyl acetate, nopyl alcohol, neobergamate, oak moss No. 1, orivone, oxyphenylon, p-cresyl methyl ether, pentalide, phenylethyl alcohol, phenylethyl acetate, phenylacetaldehyde dimethyl acetal, alpha-pinene, rubafuran, rosephenone, rose oxide, Sandalore, Sandela, Santalex, Santalinol, styrallyl acetate, styrallyl propionate, terpineol, terpinyl acetate, tetrahydrolinalool, tetrahydrolinalyl acetate, tetrahydrogeraniol, tetrahydrogeranyl acetate, tonalide, traseolide, tripral, thymol, vanillin, verdox, yara yara, anis oil, bay oil, bois de-rose oil, cananga oil, cardamon oil, cassia oil, cedarwood oil, orange oil, mandarin oil, tangerine oil, basil oil, nutmeg oil, citronella oil, clove oil, coriander oil, elemi oil, eucaryptus oil, fennel oil, galbanum oil, geranium oil, hiba oil, hinoki oil, jasmine oil, lavandin oil, lavender oil, lemon oil, lemonglass oil, lime oil, neroli oil, oak moss oil, ocotea oil, patchouli oil, peppermint oil, perilla oil, petitgrain oil, pine oil, rose oil, rosemary oil, camphor oil, palm oil, clary sage oil, sandalwood oil, spearmint oil, spike lavender oil, star anis oil, thyme oil, tonka bean tincture, turpentine oil, vanilla bean tincture, vetiver oil, bergamot oil, ylang-ylang oil, grapefruit oil, Chinese lemon (Citrus junos) oil, benzoin, Peru balsam, tolu balsam, tuberose oil, musk tincture, castrium tincture, civet tincture, and ambergris tincture.

The fragrance composition of the invention is extremely preferably contains, as a fragrant material (fragrant substance), at least one compound selected from methyl dihydrojasmonate, 10-oxa-16-hexadecanolide, styrallyl acetate, linalyl acetate, ethyl 2,2,6-trimethylcyclohexanecarboxylate, hexamethyl-1,3,4,6,7,8-hexahydrocyclopentabenzopyran, and ethylene brassylate (musk T).

The cool feeling composition or the cool feeling auxiliary composition, or the fragrance composition containing the main cool feeling substance and the cool feeling auxiliary composition (cool feeling composition) according to the invention is added to and mixed with various products which are desired to have a cool feeling effect. Examples of the products to be added and mixed with the cool feeling composition, the cool feeling auxiliary composition, and the fragrance composition of the invention include cosmetics, for example, various lotions/essences such as softening lotions, astringent lotions, cleansing lotions, calamine lotions, after shave lotions, makeup transparent lotions, and skin lotions, skin cosmetics, e.g., creams/milky lotions such as massage creams, cleansing creams, skin creams, foundation creams for summer, suntan creams, milky lotions, and lip creams, colognes such as perfumes and Colognes, lipsticks and antiperspirants; toiletry products, for example, hair cosmetics such as shampoos, conditioners, hair tonics, hair gels, and hair growth stimulants, facial cleansers, body soaps, shaving creams, shaving foams, face soaps, tooth pastes, and mouth washes; house-hold goods, for example, various bath agents such as powders, liquids, and bubbling tablets; pharmaceuticals, for example, insect repellent sprays and insect-repellent lotions, hair tonics, cataplasms, analgesic preparations (lotions, sprays, etc.), and various ointments; and foods and drinks, for example, gums, candies, ice creams, sherbets, jellies, refreshing drinks, and the like. In addition, it is also possible to utilize the compositions in moist hot hand towels capable of affording a refreshing feel in use by mixing them with a liquid preparation having a water-holding property and by impregnating fabric or the like with the resultant liquid preparation. The cosmetics, toiletry products, bath agents, pharmaceuticals, foods and drinks in the invention include all the products mentioned above. In this connection, these are merely examples of the products to be added and mixed with the cool feeling composition, cool feeling auxiliary composition, and fragrance composition of the invention. Therefore, the products to be added and mixed with the cool feeling composition, cool feeling auxiliary composition, and fragrance composition of the invention are not restricted to those specifically exemplified in the above.

The applying method and adding/mixing amount of the cool feeling composition or the cool feeling auxiliary composition or the fragrance composition containing the same according to the invention may be suitably decided depending on the kind of the product to be added and mixed with the composition or its intended purpose. Namely, as mentioned above, the cool feeling composition can be mixed at any stage during the production of the product. Alternatively, the ingredients in the cool feeling composition may be separated into the main cool feeling substance, e.g., 1-menthol and other ingredients and these may be added into the product separately, whereby, as a result, the same effects as in the case that the cool feeling composition is added into the product are obtained. Also, the cool feeling composition may be mixed with the product at suitable timing in the form of a fragrance composition to which the cool feeling composition is added beforehand. The adding amount of the cool feeling composition may be suitably decided depending on the product to be added and mixed with the cool feeling composition as mentioned above but it is usually used in a concentration of preferably 0.001 to 20% by weight, particularly 0.01 to 10% by weight based on the total composition of the product. In the case that the main cool feeling substance and the cool feeling auxiliary composition are separately added and mixed, the amounts thereof may be decided so that the total amount of the cool feeling substance and the cool feeling auxiliary composition to be added falls within the above adding range of the cool feeling composition. Furthermore, in the case of the addition in the form of the fragrance composition, the adding amount may be decided so that the amount as the cool feeling composition falls within the range mentioned above, but the fragrance composition containing the cool feeling composition is usually used in a concentration of preferably 0.01 to 20% by weight based on the total composition of the product.

The products to which the cool feeling composition, the cool feeling auxiliary composition, or the fragrance composition according to the invention is applied may contain other ingredients which constitute the products in addition to the compositions which impart a cool feeling to the products. For example, in the case of dermatological preparations for external application, such as cosmetics, toiletry products, and pharmaceuticals, depending on intended purpose, use form, and the like of the dermatological preparations for external application, powder ingredients, liquid fats and oils, solid fats and oils, waxes, hydrocarbons, higher fatty acids, higher alcohols, esters, silicones, anionic surfactants, cationic surfactants, amphoteric surfactants, nonionic surfactants, humectants, water-soluble polymers, thickeners, film-forming agents, UV absorbers, sequestering agents, lower alcohols, polyhydric alcohols, sugars, amino acids, organic amines, synthetic resin emulsions, pH adjusters, skin nourishing agents, vitamins, antioxidants, antioxidation assistants, water, and the like can be incorporated appropriately according to necessity. The dermatological preparations for external application can be produced in a usual manner using one or more of these ingredients together with the cool feeling composition, the cool feeling auxiliary composition, or the fragrance composition according to the invention.

The following will describe individual ingredients exemplified in the above more specifically. First, examples of the powder ingredients include inorganic powders, such as talc, kaolin, mica, sericite, muscovite, phlogopite, synthetic mica, lepidolite, biotite, lithia mica, vermiculite, magnesium carbonate, calcium carbonate, aluminum silicate, barium silicate, calcium silicate, magnesium silicate, strontium silicate, tungstic acid metal salts, magnesium, silica, zeolite, barium sulfate, calcined calcium sulfate (calcined gypsum), calcium phosphate, fluoroapatite, hydroxyapatite, ceramic powder, metal soaps (e.g., zinc myristate, calcium palmitate, aluminum stearate), and boron nitride; organic powders, such as polyamide resin powder (nylon powder), polyethylene powder, polymethyl methacrylate powder, polystyrene powder, styrene-acrylic acid copolymer resin powder, benzoguanamine resin powder, polytetrafluoroethylene powder, and cellulose powder; inorganic white pigments, such as titanium dioxide and zinc oxide; inorganic red pigments, such as iron oxide (rouge) and iron titanate; inorganic brown pigments, such as γ-iron oxide; inorganic yellow pigments, such as ochre and yellow iron oxide; inorganic black pigments, such as black iron oxide, carbon black, low level titanium oxide; inorganic purple pigments, such as Mango Violet and Cobalt Violet; inorganic green pigments, such as chromium oxide, chromium hydroxide, and cobalt titanate; inorganic blue pigments, such as Ultramarine and Prussian Blue; pearly pigments, such as titanium oxide-coated mica, titanium oxide-coated bismuth oxychloride, titanium oxide-coated talc, colored titanium oxide-coated mica, bismuth oxychloride, and argentine; metallic powder pigments, such as aluminum powder and copper powder; organic pigments, e.g., red Nos. 201, 202, 204, 205, 220, 226, 228, and 405, orange Nos. 203 and 204, yellow Nos. 205 and 401, and blue No. 404; zirconium, barium or aluminum lake organic pigments, e.g., red Nos. 3, 104, 106, 227, 230, 401, and 505, orange No. 205, yellow Nos. 4, 5, 202, and 203, green No. 3, and blue No. 1; and natural pigments, such as chlorophyll and β-carotene.

Examples of the liquid fats and oils include avocado oil, camellia oil, turtle oil, macadamia nut oil, corn oil, mink oil, olive oil, rape seed oil, egg yolk oil, sesame oil, persic oil, wheat germ oil, sasanqua oil, castor oil, linseed oil, safflower oil, cotton seed oil, perilla oil, soybean oil, peanut oil, tea seed oil, kaya oil, rice bran oil, Chinese tung oil, Japanese tung oil, jojoba oil, germ oil, triglycerol, glycerol trioctanoate, and glycerol triisopalmitate.

Examples of the solid fats and oils include cacao butter, coconut oil, horse fat, hydrogenated coconut oil, palm oil, beef tallow, sheep tallow, hydrogenated beef tallow, palm kernel oil, lard, beef bone fat, Japan wax kernel oil, hydrogenated oils, neat's foot oil, Japan wax, and hydrogenated castor oil.

Examples of the waxes include beeswax, candelilla wax, cotton wax, carnauba wax, bayberry wax, mealybug wax, whale wax, bran wax, lanolin, kapok wax, lanolin acetate, liquid lanolin, cane wax, lanolin fatty acid isopropyl, hexyl laurate, reduced lanolin, jojoba wax, hard lanolin, shellac wax, POE lanolin alcohol ether, POE lanolin alcohol acetate, POE cholesterol ether, lanolin fatty acid polyethylene glycol, and POE hydrogenated lanolin alcohol ether.

Examples of the hydrocarbon oils include liquid paraffin, ozokerite, squalene, pristane, paraffin, ceresine, squalene, vaselin, and microcrystalline wax.

Examples of the higher fatty acids include lauric acid, myristic acid, palmitic acid, stearic acid, behenic acid, oleic acid, 12-hydroxystearic acid, undecylenic acid, tolic acid, isostearic acid, linoleic acid, linolenic acid, eicosapentaenoic acid (EPA), and docosahexaenoic acid (DHA).

Examples of the higher alcohols include linear alcohols, such as lauryl alcohol, cetyl alcohol, stearyl alcohol, behenyl alcohol, myristyl alcohol, oleyl alcohol, and cetostearyl alcohol, and branched alcohols, such as monostearyl glycerol ether (batyl alcohol), 2-decyltetradecinol, lanolin alcohol, cholesterol, phytosterol, hexyldodecanol, isostearyl alcohol, and octyldodecanol.

Examples of the synthetic ester oils include isopropyl myristate, cetyl octanoate, octyldodecyl myristate, isopropyl palmitate, butyl stearate, hexyl laurate, myristyl myristate, decyl oleate, hexyldecyl dimethyloctanoate, cetyl lactate, myristyl lactate, lanolin acetate, isocetyl stearate, isocetyl isostearate, cholesteryl 12-hydroxystearate, ethylene glycol di-2-ethylhexylate, dipentaerythritol fatty acid esters, N-alkyl glycol stearate, neopentyl glycol dicaprinate, diisostearyl malate, glycerol di-2-heptylundecanoate, trimethylolpropane tri-2-ethylhexylate, trimethylolpropane triisostearate, pentaerythritol tetra-2-ethylhexylate, glycerol tri-2-ethylhexylate, trimethylolpropane triisostearate, cetyl 2-ethylhexanoate, 2-ethylhexyl palmitate, glycerol trimyristate, glycerol tri-2-heptylundecanoate, castor oil fatty acid methyl ester, oleic acid oil, cetostearyl alcohol, acetoglyceride, 2-heptylundecyl palmitate, diisobutyl adipate, 2-octyldodecyl N-lauryol-L-glutamate, di-2-heptylundecyl adipate, ethyl laurate, di-2-heptylundecyl sebacate, 2-hexyldecyl myristate, 2-hexyldecyl palmitate, 2-hexyldecyl adipate, diisopropyl sebacate, 2-ethylhexyl succinate, ethyl acetate, butyl acetate, amyl acetate, and triethyl citrate.

Examples of the silicones include linear polysiloxanes such as dimethyl polysiloxane, methyl phenyl polysiloxane, and methyl hydrogen polysiloxane, cyclic polysiloxanes such as decamethyl polysiloxane, dodecamethyl polysiloxane, and tetramethyl tetrahydrogen polysiloxane; silicone resins; and silicone rubbers.

Examples of the anionic surfactants include fatty acid soaps, such as soap bases, sodium laurate, and sodium palmitate; higher alkyl sulfuric ester salts, such as sodium lauryl sulfate and potassium lauryl sulfate; alkyl ether sulfuric ester salts, such as triethanolamine POE lauryl sulfate and sodium POE lauryl sulfate; N-acylsarcosinic acids, such as sodium lauroylsarcosinate, higher fatty acid amidosulfonates, such as sodium N-myristoyl-N-methyltaurine, sodium palm oil fatty acid methyltauride, and sodium laurylmethyltauride; phosphoric ester salts, such as sodium POE oleyl ether phosphate and POE stearyl ether phosphate; sulfosuccinates, such as sodium di-2-ethylhexylsulfosuccinate, sodium monolauroylmonoethanolamide polyoxyethylene sulfosuccinate, and sodium lauryl polypropylene glycol sulfosuccinate; alkylbenzenesulfonates, such as sodium linear dodecylbenzenesulfonate, triethanolamine linear dodecylbenzenesulfonate, and linear dodecylbenzenesulfonic acid; N-acylglutamates, such as monosodium N-lauroylglutamate, disodium N-stearoylglutamate, monosodium N-myristoyl-L-glutamate; higher fatty acid ester sulfuric ester salts, such as sodium hydrogenated coconut oil fatty acid glycerol sulfate; sulfated oils, such as Turkey red oil; POE alkyl ether carboxylic acids, POE alkyl allyl ether carboxylates, α-olefin sulfonates, higher fatty acid ester sulfonates, secondary alcohol sulfuric acid ester salts, higher fatty acid alkylolamide sulfuric acid ester salts, sodium lauroyl monoethanolamide succinate, ditriethanolamine N-palmitoylaspartate, and sodium casein. In the above, POE means a polyoxyethylene chain.

Examples of the cationic surfactants include alkyltrimethylammonium salts, such as stearyltrimethylammonium chloride and lauryltrimethylammonium chloride; dialkyldimethylammonium salts, such as distearyldimethylammonium chloride; poly(N,N'-dimethyl-3,5-methylenepiperidinium) chloride; alkylpyridinium salts, such as cetylpyridinium chloride; alkyl quaternary ammonium salts, alkyldimethylbenzylammonium salts, alkylisoquinolinium salts, dialkylmorpholinium salts, POE alkylamines, alkylamine salts, polyamine fatty acid derivatives, amyl alcohol fatty acid derivatives, benzalkonium chloride, and benzethonium chloride.

Examples of the amphoteric surfactants include imidazoline amphoteric surfactants, such as sodium 2-undecyl-N,N,N-(hydroxyethylcarboxymethyl)-2-imidazoline and disodium 2-cocoyl-2-imidazolinium hydroxide-1-carboxyethyloxy; and betaine surfactants, such as 2-heptadecyl-N-carboxymethyl-N-hydroxyethylimidazolinium betaine, lauryldimethylaminoacetic acid betaine, alkyl betaines, amide betaines, and sulfo betaines.

Examples of lipophilic nonionic surfactants include sorbitan fatty acid esters, such as sorbitan monooleate, sorbitan monoisostearate, sorbitan monolaurate, sorbitan monopalmitate, sorbitan monostearate, sorbitan sesquioleate, sorbitan trioleate, diglycerol sorbitan penta-2-ethylhexylate, and diglycerol sorbitan tetra-2-ethylhexylate; glycerol or polyglycerol fatty acids esters, such as glycerol mono-cotton seed oil fatty acid ester, glycerol monoerucate, glycerol sesquioleate, glycerol monostearate, glycerol α,α'-oleate pyroglutamate, and glycerol monostearate malate; propylene glycol fatty acid esters, such as propylene glycol monostearate; hydrogenated castor oil derivatives, and glycerol alkyl ethers.

Examples of hydrophilic nonionic surfactants include POE (polyoxyethylene) sorbitan fatty acid esters, such as POE sorbitan monooleate, POE sorbitan monostearate, POE sorbitan monooleate, and POE sorbitan tetraoleate; POE sorbitol fatty acid esters, such as POE sorbitol monolaurate, POE sorbitol monooleate, POE sorbitol pentaoleate, and POE sorbitol monostearate; POE glycerol fatty acid esters, such as POE glycerol monostearate, POE glycerol monoisostearate, and POE glycerol triisostearate; POE fatty acid esters, such as POE monooleate, POE distearate, POE monodioleate, and ethylene glycol distearate; POE alkyl ethers, such as POE lauryl ether, POE oleyl ether, POE stearyl ether, POE behenyl ether, POE 2-octyldodecyl ether, and POE cholestanol ether; POE alkylphenyl ethers, such as POE octylphenyl ether, POE nonylphenyl ether, and POE dinonylphenyl ether; Pluronic type surfactants, such as Pluronic; POE.POP (polyoxypropylene) alkyl ethers, such as POE.POP cetyl ether, POE.POP 2-decyltetradecyl ether, POE.POP monobutyl ether, POE.POP hydrogenated lanolin, and POE-POP glycerol ether; tetraPOE.tetraPOP ethylenediamine condensates, such as Tetronic; POE castor oil or hydrogenated castor oil derivatives, such as POE castor oil, POE hydrogenated castor oil, POE hydrogenated castor oil monoisostearate, POE hydrogenated castor oil triisostearate, POE hydrogenated castor oil monopyroglutamate monoisostearate diester, and POE hydrogenated castor oil maleate; POE beeswax lanolin derivatives, such as POE sorbitol beeswax; alkanolamides, such as coconut oil fatty acid diethanolamide, lauric acid monoethanolamide, and fatty acid isopropanolamides; POE propylene glycol fatty acid esters, POE alkylamines, POE fatty acid amides, sucrose fatty acid esters, POE nonylphenyl formaldehyde condensates; alkylethoxydimethylamine oxides; and trioleyl phosphate. In the above, POP means a polyoxypropylene chain. In addition, POE.POP means a polyoxyethylene-polyoxypropylene block copolymer chain.

Examples of the humectants include polyethylene glycol, propylene glycol, glycerol, 1,3-butylene glycol, xylitol, sorbitol, maltitol, chondroitin sulfate, hyaluronic acid, mucoitin sulfate, charonin acid, atelocollagen, cholesteryl 12-hydroxystearate, sodium lactate, bile acid salts, dl-pyrrolidonecarboxylic acid salts, short-chain soluble collagen, diglycerol (EO) PO adducts, extract of chestnut rose (R. roxburghii plena), extract of yarrow (Achillea milleofolium), and extract of melilot, ceramids, and glucosylceramids. In the above, EO represents ethylene oxide and PO represents propylene oxide.

Examples of natural water-soluble polymers include plant-derived polymers, such as gum arabic, tragacanth gum, guar gum, galactan, carob gum, gum karaya, carrageenan, pectin, agar, quince seed (quince), algae colloid (brown algae extract), starch (rice, corn, potato, wheat), and glycyrrhizinate; microorganism-derived polymers, such as xanthan gum, dextran, succinoglucan, and pullulan; and animal-derived polymers, such as collagen, casein, albumin, and gelatin.

Examples of semi-synthetic water-soluble polymers include starch polymers, such as carboxymethyl starch and methylhydroxypropyl starch; cellulose polymers, such as methyl cellulose, nitro cellulose, ethyl cellulose, methylhydroxypropyl cellulose, hydroxyethyl cellulose, sodium cellulose sulfate, hydroxypropyl cellulose, sodium carboxymethyl cellulose (CMC), crystalline cellulose, and cellulose powder; and alginate polymers, such as sodium alginate and propylene glycol alginate.

Examples of synthetic water-soluble polymers include vinyl polymers, such as polyvinyl alcohol, polyvinyl methyl ether, polyvinylpyrrolidone, and carboxyvinyl polymer (Carbopol); polyoxyethylene polymers, such as polyethylene glycol 20,000, 4,000,000 or 600,000; copolymerized polymers, such as polyoxyethylene-polypropylene copolymers; acrylic polymers, such as sodium polyacrylate, polyethyl acrylate and polyacrylamide; polyethyleneimine, and cationic polymers.

Examples of inorganic water-soluble polymers include bentonite, aluminum magnesium silicate (bee gum), Laponite, hectorite, and silicic anhydride.

Examples of the thickeners include gum arabic, carrageenan, gum karaya, tragacanth gum, carob gum, quince seed (quince), casein, dextrin, gelatin, sodium pectate, sodium alginate, methyl cellulose, ethyl cellulose, CMC, hydroxyethyl cellulose, hydroxpropyl cellulose, PVA, PVM, PVP, sodium polyacrylate, carboxyvinyl polymer, locust bean gum, guar gum, tamarind gum, dialkyldimethylammonium sulfate cellulose, xanthan gum, aluminum magnesium silicate, bentonite, and hectorite.

Examples of the UV absorbers include benzoic acid UV-absorbers, such as p-aminobenzoic acid (hereinafter abbreviated as PABA), PABA monoglycerol ester, N,N-dipropoxy-PABA ethyl ester, N,N-diethoxy-PABA ethyl ester, N,N-dimethyl-PABA ethyl ester, N,N-dimethyl-PABA butyl ester, and N,N-dimethyl-PABA methyl ester; anthranilic acid UV absorbers, such as homomenthyl N-acetylanthranilate; cinnamate UV absorbers, such as octyl cinnamate, ethyl-4-isopropylcinnamate-, methyl-2,5-diisopropylcinnamate, ethyl-2,4-diisopropylcinnamate, methyl-2,4-diisopropylcinnamate, propyl-p-methoxycinnamate, isopropyl-p-methoxycinnamate, isoamyl-p-methoxycinnamate, octyl-p-methoxycinnamate (2-ethylhexyl-p-methoxycinnamate, 2-ethoxyethyl-p-methoxycinnamate, cyclohexyl-p-methoxycinnamate, ethyl-α-cyano-β-phenylcinnamate, 2-ethylhexyl-α-cyano-β-phenylcinnamate, and glycerol mono-2-ethylhexanoyl-di-p-methoxycinnamate; benzophenone UV absorbers, such as 2,4-dihydroxybenzophenone, 2,2'-dihydroxy-4-methoxybenzophenone, 2,2'-dihydroxy-4,4'-dimethoxybenzophenoen, 2,2',4,4'-tetrahydroxybenzophenone, 2-hydroxy-4-methoxybenzophenone, 2-hydroxy-4-methoxy-4'-methylbenzophenone, 2-hydroxy-4-methoxybenzophenone-5-sulfonate salts, 4-phenylbenzophenone, 2-ethylhexyl-4'-phenylbenzophenone-2-carboxylates, 2-hydroxy-4-n-octoxybenzophenone, and 4-hydroxy-3-carboxybenzophenone; 3-(4'-methylbenzylidene)-d,l-camphor, 3-benzylidene-d,l-camphor, urocanic acid, ethyl urocanate, 2-phenyl-5-methylbenzoxazole, 2,2'-hydroxy-5-methylphenylbenzotriazole, 2-(2'-hydroxy-5'-t-octylphenyl)benzotriazole, 2-(2'-hydroxy-5'-methylphenyl)benzotriazole, dibenzalazine, dianisoylmethane, 4-methoxy-4'-t-butyldibenzoylmethane, and 5-(3,3-dimethyl-2-norbornylidene)-3-pentan-2-one.

Examples of the sequestering agents include 1-hydroxyethane-1,1-diphosphonic acid, tetrasodium 1-hydroxyethane-1,1-diphosphonate, disodium edetate, trisodium edetate, tetrasodium edetate, sodium citrate, sodium polyphosphate, sodium metaphosphate, gluconic acid, phosphoric acid, citric acid, ascorbic acid, succinic acid, and edetic acid.

The lower alcohols include methanol, ethanol, propanol, isopropanol, isobutyl alcohol, and t-butyl alcohol.

Examples of the polyhydric alcohols include dihydric alcohols, such as ethylene glycol, propylene glycol, trimethylene glycol, 1,2-butylene glycol, 1,3-butylene glycol, tetramethylene glycol, 2,3-butylene glycol, pentamethylene glycol, 2-butene-1,4-diol, hexylene glycol, and octylene glycol; trihydric alcohols, such as glycerol, trimethylolpropane, and 1,2,6-heanetriol; tetrahydric alcohols, such as pentaerythritol; pentahydric alcohols, such as xylitol, hexahydric alcohols, such as sorbitol and mannitol; polyhydric alcohol polymers, such as diethylene glycol, dipropylene glycol, triethylene glycol, polypropylene glycol, tetraethylene glycol, diglycerol, polyethylene glycol, triglycerol, tetraglycerol, and polyglycerol; dihydric alcohol alkyl ethers, such as ethylene glycol monomethyl ether, ethylene glycol monoethyl ether, ethylene glycol monobutyl ether, ethylene glycol monophenyl ether, ethylene glycol monohexyl ether, ethylene glycol mono-2-methylhexyl ether, ethylene glycol isoamyl ether, ethylene glycol benzyl ether, ethylene glycol isopropyl ether, ethylene glycol dimethyl ether, ethylene glycol diethyl ether, and ethylene glycol dibutyl ether; dihydric alcohol alkyl ethers, such as diethylene glycol monomethyl ether, diethylene glycol monoethyl ether, diethylene glycol monobutyl ether, diethylene glycol dimethyl ether, diethylene glycol diethyl ether, diethylene glycol butyl ether, diethylene glycol methyl ethyl ether, triethylene glycol monomethyl ether, triethylene glycol monoethyl ether, propylene glycol monomethyl ether, propylene glycol monoethyl ether, propylene glycol monobutyl ether, propylene glycol isopropyl ether, dipropylene glycol methyl ether, dipropylene glycol ethyl ether, and dipropylene glycol butyl ether; dihydric alcohol ether esters, such as ethylene glycol monomethyl ether acetate, ethylene glycol monoethyl ether acetate, ethylene glycol monobutyl ether acetate, ethylene glycol monophenyl ether acetate, ethylene glycol diadipate, ethylene glycol disuccinate, diethylene glycol monoethyl ether acetate, diethylene glycol monobutyl ether acetate, propylene glycol monomethyl ether acetate, propylene glycol monoethyl ether acetate, propylene glycol monopropyl ether acetate, and propylene glycol monophenyl ether acetate; glycerol monoalkyl ethers, such as chimyl alcohol, selachyl alcohol, and batyl alcohol; sugar alcohols, such as sorbitol, maltitol, maltotriose, mannitol, sucrose, erythritol, glucose, fructose, starch-degraded sugars, maltose, xylitose, and reduction alcohols of starch-degraded sugars; glysolid, tetrahydrofurfuryl alcohol, POE tetrahydrofurfuryl alcohol, POP butyl ether, POP.POE butyl ether, tripolyoxypropylene glycerol ether, POP glycerol ether, POP glycerol ether phosphate, and POP.POE pentaerythritol ether.

Examples of monosaccharides include trioses, such as D-glycerylaldehyde and dihydroxyacetone; tetroses, such as D-erythrose, D-erythrulose, D-threose, and erythritol; pentoses, such as L-arabinose, D-xylose, L-lyxose, D-arabinose, D-ribose, D-riburose, D-xylulose, and L-xylulose; hexoses, such as D-glucose, D-talose, D-psicose, D-galactose, D-fructose, L-galactose, L-mannose, and D-tagatose; heptoses, such as aldoheptose, and heptulose; octoses, such as octulose; deoxysugars, such as 2-deoxy-D-ribose, 6-deoxy-L-galactose, and 6-deoxy-L-mannose; amino sugars, such as D-glucosamine, D-galactosamine, sialic acid, aminouronic acid, and muramic acid; and uronic acids, such as D-glucuronic acid, D-mannuronic acid, L-guluronic acid, D-galacturonic acid, and L-iduronic acid.

Examples of oligosaccharides include sucrose, gentianose, umbelliferose, lactose, planteose, isolychnoses, α,α-threhalose, raffinose, lychnoses, umbilicin, stachyose verbascoses.

Examples of polysaccharides include cellulose, quince seed, chondroitin sulfate, starch, galactan, dermatan sulfate, glycogen, gum arabic, heparan sulfate, hyaluronic acid, tragacanth gum, keratan sulfate, chondroitin, xanthan gum, mucoitin sulfate, guar gum, dextran, keratosufate, locust bean gum, succinoglucan, and caronic acid.

Examples of the amino acids include neutral amino acids, such as glycine, alanine, valine, leucine, isoleucine, serine, threonine, tryptophan, cystine, cysteine, methionine, proline, and hydroxyproline; acidic amino acids, such as aspartic acid, glutamic acid, asparagine, and glutamine; and basic amino acids, such as alginine, histidine, lysine, and hydroxylysine. In addition, examples of amino acid derivatives include sodium acylsarcosinates (sodium lauroylsarcosinate), acylglutamic acid salts, sodium acyl-β-alanine, glutathione, and pyrrolidonecarboxylic acid.

Examples of the organic amines include monoethanolamine, diethanolamine, triethanolamine, morpholine, triisopropanolamine, 2-amino-2-methyl-1,3-propanediol, and 2-amino-2-methyl-1-propanol.

Examples of the synthetic resin emulsions include acrylic resin emulsions, polyethyl acrylate emulsions, acrylic resin solutions, polyalkyl acrylate emulsions, and polyvinyl acetate resin emulsions.

Examples of the pH adjustors include buffers, such as lactic acid-sodium lactate and citric acid-sodium citrate.

Examples of the vitamins include vitamins A, B1, B2, B6, E and their derivatives, pantothenic acid and its derivatives, and biotin.

Examples of the antioxidants include tocopherols, dibutylhydroxytoluene, butylhydroxyanisole, and gallic acid esters.

Examples of the antioxidation assistants include phosphoric acid, citric acid, ascorbic acid, maleic acid, malonic acid, succinic acid, fumaric acid, cephalin, hexametaphosphates, phytic acid, and ethylenediaminetetraacetic acid.

The cosmetics, toiletry products, bath agents, pharmaceuticals, and foods and drinks meant in the invention include those in various forms. The adding amount of the cool feeling composition in these products depends on the kind of the products, the amount to be used at use, and the forms of preparations of the above products include aerosols, suspensions, emulsions, plasters, ointments, cataplasms, liniments, lotions, and the like, which are forms of the solvents specified in the Pharmaceutical Affairs Law, and forms such as cleansing cosmetics, hair cosmetics, basic cosmetics, aromatic cosmetics, suntan/sunscreen goods, lip cosmetics, bathing cosmetics, and the like, which are specified in accordance with class approval standards of cosmetics. As mentioned above, the products to be added and mixed with the cool feeling composition, cool feeling auxiliary composition, or fragrance composition of the invention may be used in any forms of liquid, solid, powder, and aerosol. That is to say, they can be preferably employed in the products in which cool feeling compositions are hitherto to be incorporated.

### EXAMPLES

The following will describe the invention in greater detail with reference to Examples, Comparative Examples, and Test Examples but it should be understood that the invention is not construed as being limited thereto. Unless otherwise noted, "part(s)" and "%" mean "part(s) by weight" and "% by weight", respectively.

### Examples 1 and 2 and Comparative Examples 1 to 4

Cool feeling compositions of Examples 1 and 2 and Comparative Examples 1 to 4 having the compositions (parts) described in the following Table 1 were prepared.

**Table 1 Formulation of Cool Feeling Composition Solution**

| Ingredient | Example 1 | Example 2 | Comparative Example 1 | Comparative Example 2 | Comparative Example 3 | Comparative Example 4 |
|---|---|---|---|---|---|---|
| Dipropylene glycol | 55 | 60 | 60 | 60 | 60 | 60 |
| 1-Menthol | 35 | 30 | 35 | 30 | 30 | 30 |
| Isopulegol | 2 | 2 | - | 5 | - | 2.5 |
| 3- (1-Menthoxy)-propane-propane-1,2-diol | 2 | 2 | - | - | 5 | 2.5 |
| 2-Hydroxy-ethyl salicylate | 1 | 1 | - | - | - | - |

Using the above six kinds of the cool feeling compositions of Examples 1 and 2 and Comparative Examples 1 to 4, body shampoos of Examples 3 and 4 and Comparative Examples 5 to 8 were prepared in a usual manner according to the formulation shown in the following Table 2.

**Table 2 Body Shampoo Formulation**

| Ingredient | Mixing Amount (%) |
|---|---|
| Purified water | 45.10 |
| Triethanolamine | 9.00 |
| Lauric acid | 6.00 |
| Myristic acid | 9.00 |
| Lauryl disodium polyoxyethylene sulfosuccinate (1E.O.)(42%) | 10.00 |
| Alkyl(C8-16)glucoside | 8.00 |
| Glyceryl laurate | 1.00 |
| 2-Hydoxyethyl distearate | 2.50 |
| Coconut oil fatty acid diethanol amide | 3.00 |
| Propylene glycol | 5.00 |
| Dibutylhydroxytoluene | 0.05 |
| Disodium edetate | 0.10 |
| Ethyl p-hydroxybenzoate | 0.20 |
| Methyl p-hydroxybenzoate | 0.10 |
| Cool feeling composition | 0.95 |
| Total | 100.00 |

### Test Example 1

Each of the body shampoos of Examples 3 and 4 and Comparative Examples 5 to 8 were used in a bathroom and evaluated for irritant odor-alleviating effect in use, cool feeling strength immediately after use, and cool feeling strength after 20 minutes of use (persistency). Evaluation was made by 14 fragrance expert panelists who had over 5 years experienced. An evaluation score was determined according to the following seven-stage criteria and an average value was summarized, the body shampoo of Comparative Example 5 containing 1-menthol alone being score 4.

### [Evaluation Score]

7: Very high as compared with Comparative Example 5
6: High as compared with Comparative Example 5
5: Slightly high as compared with Comparative Example 5
4: Equal to Comparative Example 5
3: Slightly low as compared with Comparative Example 5
2: Low as compared with Comparative Example 5
1: Very low as compared with Comparative Example 5

**Table 3**

| | Example 3 | Example 4 | Comparative Example 5 | Comparative Example 6 | Comparative Example 7 | Comparative Example 8 |
|---|---|---|---|---|---|---|
| Irritant odor-alleviating effect | 5.8 | 6.1 | - | 2.3 | 3.0 | 4.4 |

**Table 4**

| | Example 3 | Example 4 | Comparative Example 5 | Comparative Example 6 | Comparative Example 7 | Comparative Example 8 |
|---|---|---|---|---|---|---|
| Cool feeling strength | 6.2 | 6.0 | - | 2.7 | 2.4 | 3.8 |

**Table 5**

| | Example 3 | Example 4 | Comparative Example 5 | Comparative Example 6 | Comparative Example 7 | Comparative Example 8 |
|---|---|---|---|---|---|---|
| Cool feeling persistency | 6.5 | 6.3 | - | 3.2 | 4.2 | 4.8 |

As is apparent from the above Tables 3, 4 and 5, some improvement in irritant odor-alleviating effect and cool feeling persistency is observed in the case of mixing 1-menthol with isopulegol and 3-(1-menthoxypropane)-1,2-diol (Comparative Example 8) as compared with the case of using 1-menthol alone (Comparative Example 5) but the improvement is not yet satisfactory. However, from the evaluation results of Examples 1 and 2, it is understood that remarkably improved effects are obtained on any properties of the irritant odor-alleviating effect, cool feeling strength, and persistency by further mixing of a salicylic acid ester,.

### Examples 5 and 6 and Comparative Examples 9 to 12

Cool feeling compositions of Examples 5 and 6 and Comparative Examples 9 to 12 having the compositions (parts) described in the following Table 6 were prepared.

**Table 6**

| Ingredient | Example 5 | Example 6 | Comparative Example 9 | Comparative Example 10 | Comparative Example 11 | Comparative Example 12 |
|---|---|---|---|---|---|---|
| 1-Menthol | 30 | 30 | 35 | 30 | 30 | 30 |
| Isopulegol | 2 | 2 | - | 5 | - | 2.5 |
| 3- (1-Menthoxy)-propane-1,2-diol | 1 | 2 | - | - | 5 | 2.5 |
| 2-Hydrox-ethyl salicylate | 2 | 1 | - | - | - | - |

### Examples 7 and 8 and Comparative Examples 13 to 16

Six kinds of the cool feeling composition-containing fragrance compositions were obtained from 35 parts of each of the six kinds of the cool feeling compositions of the above Examples 5 and 6 and Comparative Examples 9 to 12 and 60 parts of a citrus herbal-note blended fragrant material (manufactured by Takasago International Corporation). Using these six kinds of the cool feeling compositions, body shampoos of Examples 7 and 8 and Comparative Examples 13 to 16 were prepared according to the formulation shown in the following Table 7.

**Table 7 Body Shampoo Formulation**

| Ingredient | Mixing Amount (%) |
|---|---|
| Purified water | 45.100 |
| Triethanolamine | 9.000 |
| Lauric acid | 6.000 |
| Myristic acid | 9.000 |
| Lauryl disodium polyoxyetylene sulfosuccinate (1E.O.)(42%) | 10.000 |
| Alkyl(C8-16)glucoside | 8.000 |
| Glyceryl laurate | 1.000 |
| 2-Hydoxyethyl distearate | 2.500 |
| Coconut oil fatty acid diethanol amide | 3.000 |
| Propylene glycol | 5.000 |
| Dibutylhydroxytoluene | 0.050 |
| Disodium edetate | 0.100 |
| Ethyl p-hydroxybenzoate | 0.200 |
| Methyl p-hydroxybenzoate | 0.100 |
| Cool feeling composition-containing blended fragrant material | 0.950 |
| Total | 100.00 |

### Test Example 2

The body shampoos of Examples 7 and 8 and Comparative Examples 13 to 16 were used in the same manner as in Test Example 1 and evaluation for fragrance diffusivity in use, irritant odor-alleviating effect, and cool feeling strength, and cool feeling persistency and fragrance-remaining property at the passage of 20 minutes after use was made. The evaluation results are shown in Tables 8 to 12.

Evaluation was made by 12 fragrance expert panelists who had over 5 years experienced. An evaluation score was determined according to the following seven-stage criteria and an average values were summarized, the body shampoo of Comparative Example 13 containing 1-menthol alone being score 4.

### [Evaluation Score]

7: Very high as compared with Comparative Example 13
6: High as compared with Comparative Example 13
5: Slightly high as compared with Comparative Example 13
4: Equal to Comparative Example 13
3: Slightly low as compared with Comparative Example 13
2: Low as compared with Comparative Example 13
1: Very low as compared with Comparative Example 13

**Table 8**

| | Example 7 | Example 8 | Comparative Example 13 | Comparative Example 14 | Comparative Example 15 | Comparative Example 16 |
|---|---|---|---|---|---|---|
| Fragrance diffusivity | 5.0 | 5.3 | - | 3.8 | 4.1 | 3.4 |

**Table 9**

| | Example 7 | Example 8 | Comparative Example 13 | Comparative Example 14 | Comparative Example 15 | Comparative Example 16 |
|---|---|---|---|---|---|---|
| Irritant odor-alleviating effect | 5.3 | 5.3 | - | 2.6 | 3.7 | 3.4 |

**Table 10**

| | Example 7 | Example 8 | Comparative Example 13 | Comparative Example 14 | Comparative Example 15 | Comparative Example 16 |
|---|---|---|---|---|---|---|
| Cool feeling strength | 5.5 | 5.6 | - | 3.6 | 4.0 | 4.1 |

**Table 11**

| | Example 7 | Example 8 | Comparative Example 13 | Comparative Example 14 | Comparative Example 15 | Comparative Example 16 |
|---|---|---|---|---|---|---|
| Cool feeling persistency | 5.2 | 5.3 | - | 3.0 | 3.6 | 4.3 |

**Table 12**

| | Example 7 | Example 8 | Comparative Example 13 | Comparative Example 14 | Comparative Example 15 | Comparative Example 16 |
|---|---|---|---|---|---|---|
| fragrance-remaining property | 4.9 | 5.6 | - | 2.7 | 4.5 | 4.2 |

As is apparent from the above Tables 8 to 12, in the case of mixing 1-menthol with isopulegol and/or 3-(1-menthoxypropane)-1,2-diol, some tendency of improvement in fragrance diffusivity and fragrance-remaining property is observed in the composition in which 3-(1-menthoxypropane)-1,2-diol has been mixed, and some tendency of improvement in cool feeling strength, cool feeling persistency, and fragrance-remaining property is observed in the composition in which isopulegol and 3-(1-menthoxypropane)-1,2-diol have been both mixed but the improvement is not yet satisfactory in every case. On the other hand, it is understood that the compositions (Examples 7 and 8) in which a salicylic acid ester (ingredient C) is further mixed with isopulegol and 3-(1-menthoxypropane)-1,2-diol (ingredient B) enable achievement of excellent effects on any of fragrance diffusivity in use, fragrance-remaining property, irritant odor-alleviating effect, cool feeling strength, and persistency.

### 9 to 11

Three kinds of cool feeling composition solutions of Examples 9 to 11 having the compositions (parts) described in the following Table 13 were prepared.

**Table 13**

| Ingredient | Example 9 | Example 10 | Example 11 | Comparative Example 1 |
|---|---|---|---|---|
| Dipropylene glycol | 60 | 60 | 60 | 60 |
| 1-Menthol | 30 | 30 | 30 | 35 |
| Isopulegol | 2 | 2 | 2 | - |
| 3-(1-Menthoxy)-propane-1,2-diol | 2 | 2 | 2 | - |
| Benzyl salicylate | 1 | - | - | - |
| Menthyl salicylate | - | 1 | - | - |
| 2-Hydroxyethyl salicylate | - | - | 1 | - |

### Test Example 3

Each of the above three kinds of the cool feeling composition solutions of Examples 9 to 11 were subjected to comprehensive evaluation for minty feeling, natural feeling, balance of smell, and sweet fragrance characteristic to menthol. Evaluation was made by 13 fragrance expert panelists who had over 5 years experienced. An evaluation score was determined according to the following seven-stage criteria and an average values were summarized, the composition of Comparative Example 1 containing 1-menthol alone being score 4. The results are shown in Table 14.

### [Evaluation Score]

7: Very excellent as compared with Comparative Example 1
6: Excellent as compared with Comparative Example 1
5: Slightly excellent as compared with Comparative Example 1
4: Equal to Comparative Example 1
3: Comparative Example 1 is slightly excellent
2: Comparative Example 1 is excellent
1: Comparative Example 1 is very excellent

**Table 14 Comprehensive Evaluation Results**

| | Example 9 | Example 10 | Example 11 |
|---|---|---|---|
| Comprehensive evaluation score | 4.8 | 5.2 | 6.1 |

From Table 14, it is understood that any of benzyl salicylate, menthyl salicylate, and 2-hydroxyethyl salicylate affords good results in fragrance, such as minty feeling, natural feeling, balance of smell, and sweet fragrance characteristic to menthol, and particularly, the 2-hydroxyethyl ester is excellent.

### Example 12 and Comparative Example 17

According to the formulation shown in Table 15, fragrance compositions of Example 12 and Comparative Example 17 were prepared in a usual manner.

**Table 15**

| | Example 12 | Example 17 |
|---|---|---|
| Apple base (manufactured by Takasago International Corporation) | 8.0 (g) | 8.0 (g) |
| Bergamot oil | 14.0 | 14.0 |
| Ethyl acetoacetate | 5.0 | 5.0 |
| Methyl dihydrojasmonate | 23.0 | 23.0 |
| Laurinal | 3.0 | 3.0 |
| Levosandol^{(R)} (manufactured by Takasago International Corporation) | 4.0 | 4.0 |
| Orange oil | 8.0 | 8.0 |
| 10-Oxa-16-hexadecanolide | 8.0 | 8.0 |
| Phenoxanol^{(R)} (manufactured by IFF) | 6.0 | 6.0 |
| Styrallyl acetate | 3.0 | 3.0 |
| Ethyl 2,2,6-trimethylcyclohexane-carboxylate | 8.0 | 8.0 |
| Cool feeling composition of Example 6 | 30.0 | - |
| 1-Menthol | - | 30.0 |

### Example 13 and Comparative Example 18

According to the formulation shown in Table 16, shampoos of Example 13 and Comparative Example 18 which contained 1.0% of the fragrance compositions of Example 12 and Comparative Example 17, respectively, were prepared each in an amount of 100 g.

**Table 16 Formulation of Shampoo**

| Ingredient | Weight (g) |
|---|---|
| Sodium polyoxyethylene lauryl ether sulfate | 14.00 |
| Lauric acid amide propyl betaine | 4.00 |
| Coconut oil fatty acid diethanolamide | 3.00 |
| Cationated cellulose | 0.50 |
| Ethylene glycol distearate | 1.00 |
| p-Hydroxybenzoic acid ester | 0.25 |
| Citric acid | Proper amount |
| Fragrance composition | 1.00 |
| Purified water | Balance |
| Total | 100.00 |

### Test Example 4

Using the above shampoos of Example 13 and Comparative Example 18, fragrance diffusivity and fragrance-remaining property of the shampoos were evaluated by the following test method. The results are shown in Tables 17 and 18.

### (Test Method)

Five grams of a tress of hair (human hair) was treated with 2.5 g of each shampoo and 5 ml of hot water (40°C) for 1 minute, rinsed with 1,000 ml of hot water (40°C), dried with a towel, and then fixed and allowed to stand on an aluminum foil to prepare an evaluation sample. With regard to the fragrance diffusivity immediately after the fixing on the foil and the fragrance-remaining property after about 5 hours had passed at room temperature, the following was evaluated: which has higher fragrance diffusivity and which has stronger fragrance-remaining property, the shampoo of Example 13 or the shampoo of Comparative Example 18. Evaluation was made by 10 fragrance expert panelists who had over 5 years experienced and was repeated three times (total 30 panelists).

**Table 17 Test Results of Fragrance Diffusivity on Shampoo**

| | Example 13 | Comparative Example 18 | No difference felt |
|---|---|---|---|
| Number of panelists | 15 | 7 | 8 |

**Table 18 Test Results of Fragrance-remaining property on Shampoo**

| | | | |
|---|---|---|---|
| | Example 13 | Comparative Example 18 | No difference felt |
| Number of panelists | 14 | 8 | 8 |

### Example 14 and Comparative Example 19

According to the formulation shown in the following Table 19, conditioners of Example 14 and Comparative Example 19 which contained 1.0% of the fragrance compositions of Example 12 and Comparative Example 17, respectively, were prepared each in an amount of 100 g.

**Table 19 Formulation of Conditioner**

| Ingredient | Weight (g) |
|---|---|
| Stearyltrimethylammonium chloride | 0.50 |
| Distearyldimethylammonium chloride | 1.50 |
| Jojoba oil | 2.50 |
| Cetanol | 4.50 |
| Liquid lanolin | 2.00 |
| Polyoxyethylene stearyl ether | 1.50 |
| Concentrated glycerol | 7.00 |
| p-Hydroxybenzoic acid ester | 0.25 |
| Sodium hydroxide | Proper amount |
| Citric acid | Proper amount |
| Fragrance composition | 1.00 |
| Purified water | Balance |
| Total | 100.00 |

### Test Example 5

Using the above shampoos of Example 14 and Comparative Example 19, fragrance diffusivity and fragrance-remaining property of the shampoos were evaluated by the following test method. The results are shown in Tables 20 and 21.

### (Test Method)

Five grams of a tress of hair (human hair) was treated with 2.5 g of each conditioner and 5 ml of hot water (40 °C) for 1 minute, rinsed with 1,000 ml of hot water (40 °C), dried with a towel, and then fixed and allowed to stand on an aluminum foil to prepare an evaluation sample. The fragrance diffusivity immediately after the fixing on the foil and the fragrance-remaining property after about 5 hours had passed at room temperature were evaluated. Evaluation was made by 10 fragrance expert panelists who had over 5 years experienced and was repeated three times (total 30 panelists).

**Table 20 Test Results of Fragrance Diffusivity on Conditioner**

| | Example 14 | Comparative Example 19 | No difference felt |
|---|---|---|---|
| Number of panelists | 13 | 9 | 8 |

**Table 21 Test Results of Fragrance-remaining property on Conditioner**

| | Example 14 | Comparative Example 19 | No difference felt |
|---|---|---|---|
| Number of panelists | 20 | 5 | 5 |

As is apparent from the results of Test Examples 4 and 5, the shampoo and conditioner which contain the blended fragrant material containing the cool feeling composition of the invention both exhibit excellent results in fragrance diffusivity and fragrance-remaining property, particularly excellent effects on fragrance-remaining property.

### Example 15

According to the formulation shown in the following Table 22, 100 g of a cream was prepared in a usual manner.

**Table 22 Formulation of Cream**

| Ingredient | Weight (%) |
|---|---|
| Hydrogenated oil | 6.00 |
| Stearic acid | 3.00 |
| Cetanol | 4.00 |
| Squalane | 2.00 |
| Neopentyl glycol dicaprate | 8.00 |
| Polyoxyethylene sorbitan monostearate (20E.O.) | 4.00 |
| Lipophilic glycerol monostearate | 2.30 |
| Stearoyl-N-methyltaurine sodium | 1.70 |
| 1,3-Butylene glycol | 7.00 |
| Concentrated glycerol | 3.00 |
| p-Hydroxybenzoic acid ester | 0.25 |
| Floral powdery fragrance composition BF-6370-C (manufactured by Takasago International Corporation) containing 50% of the cool feeling composition of Example 5 | 0.50 |
| Vanillyl butyl ether | 0.01 |
| Purified water | Balance |
| Total | 100.00 |

### Example 16

According to the formulation shown in the following Table 23, 100 g of a hair rinse was prepared in a usual manner.

**Table 23 Formulation of Hair rinse**

| Ingredient | Weight (g) |
|---|---|
| Stearyltrimethylammonium chloride | 1.00 |
| Cetanol | 3.00 |
| Methyl polysiloxane | 1.00 |
| Polyoxyethylene stearyl ether | 1.00 |
| Propylene glycol | 5.00 |
| p-Hydroxybenzoic acid ester | 0.25 |
| Sodium hydroxide | Proper amount |
| Citric acid | Proper amount |
| Floral powdery fragrance composition BF-6372 (manufactured by Takasago International Corporation) containing 30% of the cool feeling composition of Example 5 | 1.00 |
| Purified water | Balance |
| Total | 100.00 |

### Example 17

According to the formulation shown in the following Table 24, 100 g of a hair tonic was prepared in a usual manner.

**Table 24 Formulation of Hair Tonic**

| Ingredient | Weight (g) |
|---|---|
| Swertia herb extract | 2.00 |
| Cool feeling composition in which 1-menthol:isopulegol:2-(1-menthoxy)ethan-1-ol:2-hydroxyethyl salicylate is 5:2:2:1 (weight ratio) | 0.20 |
| Hinokitiol | 0.01 |
| Citrus fuzea fragrance composition BF-6040 (manufactured by Takasago International Corporation) | 0.20 |
| p-Hydroxybenzoic acid ester | 0.20 |
| Polyoxyethylene hydrogenated castor oil | 0.50 |
| Purified water | Balance |
| Total | 100.00 |

### Example 18

According to the formulation shown in the following Table 25, 100 g of a deodorant powder spray was prepared in a usual manner.

**Table 25 Formulation of Deodorant Powder Spray**

| Ingredient | Weight (%) |
|---|---|
| Chlorohydroxyaluminum | 1.00 |
| Silicic anhydride | 1.00 |
| Isopropyl myristate | 2.00 |
| Octamethylcyclotetrasiloxane | 2.00 |
| Triclosan | 0.02 |
| Sorbitan sesquioleate | 0.10 |
| 1-Menthol | 0.30 |
| Cool feeling composition in which N-ethyl-1-menthylcarboxamide:3-(1-menthoxy)propane-1,2-diol:phenethyl salicylate is 5:3:2 (weight ratio) | 0.50 |
| Vanillyl butyl ether | 0.05 |
| Citrus floral fragrance composition BF-6021 (manufactured by Takasago International Corporation) | 0.30 |
| 95% Ethanol | 3.33 |
| LPG | Balance |
| Total | 100.00 |

### Example 19

According to the formulation shown in the following Table 26, 100 g of a liquid bath agent was prepared in a usual manner.

**Table 26 Formulation of Liquid Bath Agent**

| Ingredient | Weight (g) |
|---|---|
| Dipropylene glycol | 50.00 |
| 1,3-Butylene glycol | 10.00 |
| p-Hydroxybenzoic acid ester | 0.20 |
| Cool feeling composition in which 1-menthol:isopulegol:2-methyl-3-(1-menthoxy)propane-1,2-diol:1-menthyl lactate:1-menthyl salicylate is 5:2:1:1:1 (weight ratio) | 0.30 |
| Lemon fragrance composition BF-6032 (manufactured by Takasago International Corporation) | 1.00 |
| Purified water | Balance |
| Total | 100.00 |

### Example 20

According to the formulation shown in the following Table 27, 100 g of a toothpaste was prepared in a usual manner.

**Table 27 Formulation of Toothpaste**

| Ingredient | Weight (g) |
|---|---|
| Cool feeling composition of Example 6 | 0.50 |
| Calcium hydrogen phosphate (dihydrate) | 50.00 |
| Glycerol | 25.00 |
| Sodium lauryl sulfate | 1.40 |
| Carboxymethyl cellulose sodium | 1.50 |
| Saccharin sodium | 0.20 |
| Sodium benzoate | 0.10 |
| Strawberry type flavor ZX-3687 (manufactured by Takasago International Corporation) | 0.70 |
| Purified water | Balance |
| Total | 100.00 |

When individual products of Examples 15 to 20 were evaluated for cool feeling strength, persistency, and an irritant odor-alleviating effect, remarkably good results were obtained and also remarkably improved fragrance diffusivity, natural feeling, balance of smell, sweetness, and fragrance-remaining property were observed in all cases as compared with the case of 1-menthol alone and the cases of mixing 1-menthol and isopulegol, 3-(1-menthoxypropane)-1,2-diol, 2-(1-menthoxy)ethan-1-ol, 2-methyl-3-(1-menthoxy)propane-1,2-diol, N-ethyl-1-menthylcarboxamide, 1-menthyl lactate, or the like.

### Industrial Applicability

As mentioned above, the cool feeling composition of the invention exhibits remarkably improved effects in fragrance, such as minty feeling, natural feeling, balance of smell, and sweet fragrance characteristic to menthol, and an irritant odor-alleviating effect. Moreover, the cool feeling composition can impart excellent effects in view of cool feeling strength and persistency. Furthermore, the cool feeling composition of the invention exhibits an effect of enhancing fragrance diffusivity and fragrance-remaining property of a blended fragrant material and the effect can be also realized in product forms.

## Claims

1. A cool feeling composition, which comprises a cool feeling substance comprising (A) 1-menthol and (B) at least one compound selected from isopulegol, 3-(1-menthoxy)propane-1,2-diol, 2-(1-menthoxy)ethane-1-ol, 3-(1-menthoxy)propane-1-ol, 2-methyl-3-(1-menthoxy)propane-1,2-diol, N-ethyl-1-menthylcarboxamide, p-menthane-3,8-diol, 1-menthyl lactate, and 2-isopropyl-N,2,3-trimethylbutanamide, and (C) a salicylic acid ester represented by the following general formula (I), and having composition ratios of the ingredient (A) to the ingredient (B), the ingredient (B) to the ingredient (C), and the ingredient (A) to the ingredient (C).by weight being 95:5 to 30:70, 90:10 to 30:70, and 99.5:0.5 to 30:70, respectively: 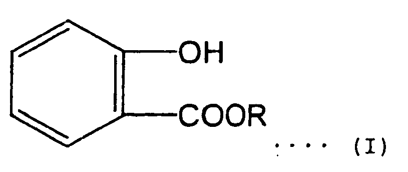 wherein R represents a 2-hydroxyethyl group, a 2,3-dihydroxypropyl group, a benzyl group, a phenethyl group or a menthyl group.

2. The cool feeling composition according to claim 1, wherein the cool feeling substances of the ingredient (B) are isopulegol and 3-(1-menthoxy)propane-1,2-diol and the salicylic acid ester of the ingredient (C) is 2-hydroxyethyl salicylate.

3. Use of a composition, which comprises (B) at least one compound selected from isopulegol, 3-(1-menthoxy)propane-1,2-diol, 2-(1-menthoxy)ethane-1-ol, 3-(1-menthoxy)propane-1-ol, 2-methyl-3-(1-menthoxy)propane-1,2-diol, N-ethyl-1-menthylcarboxamide, p-menthane-3,8-diol, 1-menthyl lactate, and 2-isopropyl-N,2,3-trimethylbutanamide, (C) at least one salicylic acid ester represented by the general formula (I) according to claim 1, and, according to necessity, (A) 1-menthol, and having composition ratios of the ingredient (A) to the ingredient (B), the ingredient (B) to the ingredient (C), and the ingredient (A) to the ingredient (C) by weight being 0:100 to 20:80, 90:10 to 30: 70, and 0:100 to 20: 80, respectively for the preparation of a product for producing a cool feeling effect.

4. A fragrance composition, wherein the cool feeling composition according to claim 1 or 2 is mixed in an amount of 0.01 to 90% by weight.

5. A food and drink, cosmetic, toiletry product, bath agent, or pharmaceutical, wherein the cool feeling composition according to claim 1 or 2 is mixed in an amount of 0.001 to 20% by weight.

6. Use according to claim 3, wherein the composition is admixed with 1-menthol in a food and drink, cosmetic, toiletry product, bath agent, or pharmaceutical.

7. A food and drink, cosmetic, toiletry product, bath agent, or pharmaceutical, which contains the fragrance composition according to claim 4 in an amount of 0.01 to 20% by weight.

8. Use of the cool feeling composition according to claim 1 or 2 for a food and drink, cosmetic, toiletry product, bath agent, or pharmaceutical.

9. The composition of claim 1, wherein compound (C) is 2-hydroxyethyl salicylate.

10. Use according to claim 3, wherein compound (C) is 2-hydroxyethyl salicylate.

## Patentansprüche

1. Sich kühl anfühlende Zusammensetzung, die eine sich kühl anfühlende Substanz umfasst, umfassend (A) 1-Menthol und (B) zumindest eine Verbindung ausgewählt aus Isopulegol, 3-(1-Menthoxy)propan-1,2-diol, 2-(1-Menthoxy)ethan-1-ol, 3-(1-Menthoxy)propan-1-ol, 2-Methyl-3-(1-menthoxy)propan-1,2-diol, N-ethyl-1-menthylcarboxamid, p-Menthan-3,8-diol, 1-Menthyllactat, und 2-Isopropyl-N,2,3-trimethylbutanamid und (C) einen Salizylsäureester repräsentiert durch die nachfolgende allgemeine Formel (I) und mit Zusammensetzungsverhältnissen des Inhaltsstoffs (A) zum Inhaltsstoff (B), des Inhaltsstoffs (B) zu Inhaltsstoff (C), und des Inhaltsstoffs (A) zu Inhaltsstoff (C) nach Gewicht von 95:5 bis 30:70, 90:10 bis 30:70, und 99,5:0,5 bis 30:70: 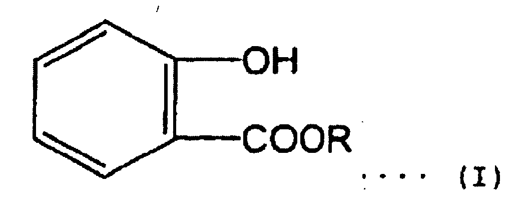 wobei R eine 2-Hydroxyethylgruppe, eine 2,3-Dihydroxypropylgruppe, eine Benzylgruppe, eine Phenethylgruppe oder eine Menthylgruppe repräsentiert.

2. Sich kühl anfühlende Zusammensetzung nach Anspruch 1, wobei die sich kühl anfühlenden Substanzen des Inhaltsstoffs (B) Isopulegol und 3-(1-Menthoxy)propan-1,2-diol sind und der Salizylsäureester des Inhaltsstoffs (C) 2-Hydroxyethylsalizylat ist.

3. Verwendung einer Zusammensetzung, die (B) zumindest eine Verbindung umfasst, ausgewählt aus Isopulegol, 3-(1-Menthoxy)propan-1,2-diol, 2-(1-Mentoxy)ethan-1-ol, 3-(1-Menthoxy)propan-1-ol, 2-Methyl-3-(1-Menthoxy)propan-1,2-diol, N-ethyl-1-menthylcarboxamid, p-Menthan-3,8-diol, 1-Menthyllactat, und 2-Isopropyl-N,2,3-trimethylbutanamid, (C) zumindest einen Salizylsäureester, repräsentiert durch die allgemeine Formel (I) nach Anspruch 1 und nach Bedarf, (A) 1-Menthol und mit Zusammensetzungsverhältnissen des Inhaltsstoffs (A) zum Inhaltsstoff (B), des Inhaltsstoffs (B) zum Inhaltsstoff (C) und des Inhaltsstoffs (A) zum Inhaltsstoff (C) nach Gewicht von 0:100 bis 20:80, 90:10 bis 30:70 und 0:100 bis 20:80 zur Herstellung eines Produktes zur Erzeugung eines sich kühl anfühlenden Effektes.

4. Duftstoffzusammensetzung, wobei die sich kühl anfühlende Zusammensetzung nach Anspruch 1 oder 2 in einer Menge von 0,01 bis 90 Gew.-% vermischt wird.

5. Nahrungsmittel und Getränk, Kosmetikum, Toilettenprodukt, Bademittel oder pharmazeutisches Mittel, wobei die sich kühl anfühlende Zusammensetzung nach Anspruch 1 oder 2 in einer Menge von 0,001 bis 20 Gew.-% vermischt wird.

6. Verwendung nach Anspruch 3, wobei die Zusammensetzung mit 1-Menthol in einem Nahrungsmittel und Getränk, Kosmetikum, Toilettenprodukt, Bademittel oder Pharmazeutikum vermischt wird.

7. Nahrungsmittel oder Getränk, Kosmetikum, Toilettenartikel, Bademittel oder pharmazeutisches Mittel, das die Duftstoffzusammensetzung nach Anspruch 4 in einer Menge von 0,01 bis 20 Gew.-% enthält.

8. Verwendung der sich kühl anfühlenden Zusammensetzung nach Anspruch 1 oder 2 für ein Nahrungsmittel und Getränk, Kosmetikum, Toilettenartikel, Bademittel oder pharmazeutisches Mittel.

9. Zusammensetzung nach Anspruch 1, wobei Bestandteil (C) 2-Hydroxyethylsalizylat ist.

10. Verwendung nach Anspruch 3, wobei Bestandteil (C) 2-Hydroxyethylsalizylat ist.

## Revendications

1. Composition à sensation de fraîcheur, qui comprend une substance à sensation de fraîcheur comprenant (A) du 1-menthol et (B) au moins un composé choisi parmi l'isopulégol, le 3-(1-menthoxy)propane-1,2-diol, le 2-(1-menthoxy)éthane-1-ol, le 3-(1-menthoxy)propane-1-ol, le 2-méhyl-3-(1-menthoxy)propane-1,2-diol, le N-éthyl-1-menthylcarboxamide, le p-menthane-3,8-diol, le lactate de 1-menthyle et le 2-isopropyl-N,2,3-triméthylbutanamide, et (C) un ester d'acide salicylique représenté par la formule générale (I) suivante, et ayant des rapports de composition de l'ingrédient (A) sur l'ingrédient (B), de l'ingrédient (B) sur l'ingrédient (C), et de l'ingrédient (A) sur l'ingrédient (C) en poids qui sont de 95:5 à 30:70, de 90:10 à 30:70, et de 99,5:0,5 à 30:70, respectivement : 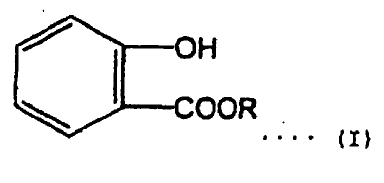 dans laquelle R représente un groupe 2-hydroxyéthyle, un groupe 2,3-dihydroxypropyle, un groupe benzyle, un groupe phénéthyle ou un groupe menthyle.

2. Composition à sensation de fraîcheur selon la revendication 1, dans laquelle les substances à sensation de fraîcheur de l'ingrédient (B) sont l'isopulégol et le 3-(1-menthoxy)propane-1,2-diol et l'ester d'acide salicylique de l'ingrédient (C) et le salicylate de 2-hydroxyéthyle.

3. Utilisation d'une composition, qui comprend (B) au moins un composé choisi parmi l'isopulégol, le 3-(1-menthoxy)propane-1,2-diol, le 2-(1-menthoxy)éthane-1-ol, le 3-(1-menthoxy)propane-1-ol, le 2-méhyl-3-(1-menthoxy)propane-1,2-diol, le N-éthyl-1-menthylcarboxamide, le p-menthane-3,8-diol, le lactate de 1-menthyle et le 2-isopropyl-N,2,3-triméthylbutanamide, et (C) au moins un ester d'acide salicylique représenté par la formule générale (I) selon la revendication 1, selon la nécessité, (A) du 1-menthol, et ayant des rapports de composition de l'ingrédient (A) sur l'ingrédient (B), de l'ingrédient (B) sur l'ingrédient (C), et de l'ingrédient (A) sur l'ingrédient (C) en poids qui sont de 0 : 100 à 20 : 80, de 90 : 10 à 30 : 70, et de 0 : 100 à 20 : 80, respectivement, pour la préparation d'un produit destiné à produire un effet de sensation de fraîcheur.

4. Composition de parfum, dans laquelle la composition à sensation de fraîcheur selon la revendication 1 ou 2 est mélangée en une quantité de 0,01 à 90 % en poids.

5. Aliment et boisson, produit cosmétique, produit de toilette, agent de bain, ou produit pharmaceutique, dans lequel la composition à sensation de fraîcheur, selon la revendication 1 ou 2, est mélangée en une quantité de 0,001 à 20 % en poids.

6. Utilisation selon la revendication 3, dans laquelle la composition est mélangée à du 1-menthol dans un aliment et une boisson, un produit cosmétique, un produit de toilette, un agent pour le bain ou un produit pharmaceutique.

7. Aliment et boisson, produit cosmétique, produit de toilette, agent pour le bain, ou produit pharmaceutique, qui contient la composition de parfum selon la revendication 4 en une quantité de 0,01 à 20 % en poids.

8. Utilisation de la composition à sensation de fraîcheur selon la revendication 1 ou 2 pour un aliment et une boisson, un produit cosmétique, un produit de toilette, un agent pour le bain ou un produit pharmaceutique.

9. Composition selon la revendication 1, dans laquelle le composant (C) est le salicylate de 2-hydroxyéthyle.

10. Utilisation selon la revendication 3, dans laquelle le composant (C) est le salicylate de 2-hydroxyéthyle.
